# EUROPEAN PATENT APPLICATION

(11) **EP 1 454 625 A1**
(43) Date of publication of application: **08.09.2004**
(21) Application number: 03100549.9
(22) Date of filing: 06.03.2003
(51) Int. Cl.: A61K 31/506, A61P 13/12

(54) **Pyridylsulfonamidyl-pyrimidines for the treatment of diabetic nephropathies**

(71) Applicant: Speedel Development AG, 4051 Basel (CH)
(72) Inventor: The designation of the inventor has not yet been filed
(74) Representative: Maué, Paul Georg

(57) **Abstract**

The present invention relates to the use of a compound of formula (I) 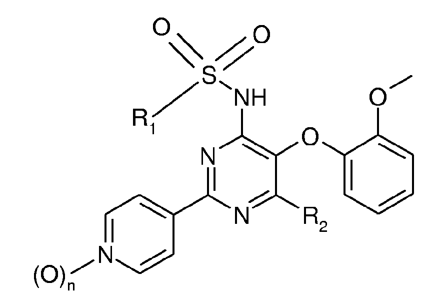 wherein
R₁ is pyridyl or thiazolyl, any of which may optionally be substituted with C₁₋₈alkyl or C₂₋₈alkenyl ; and
a) R₂ is methoxy and n is zero or one; or
b) R₂ is chlorine and n is zero
and pharmaceutically acceptable salts thereof
for the treatment of diabetic nephropathy.

## Description

The present invention relates to a new medicament/method for the treatment of diabetic nephropathy comprising the use of specific pyridylsulfonamido pyrimidines.

Diabetic nephropathy is the principle cause of end stage renal disease in the western world. It is a major cause of morbidity and mortality in Type-I Diabetes, but is an increasing problem in Type-II Diabetes and because the incidence of this is five times that of Type-I Diabetes, it contributes at least 50% of diabetics with end stage renal disease.

The initial stage of subtle morphologic changes in the renal glomeruli is followed by microalbuminuria. This is associated with a modestly rising blood pressure and an increased incidence of cardiovascular disease. There follows a continued increase in urinary protein excretion and declining glomerular filtration rate. Diabetic nephropathy has many possible underlying pathophysiological causes including metabolic, glycosylation of proteins, haemodynamics, altered flow/pressure in glomeruli, the development of hypertension and cytokine production; all of these are associated with the development of extracellular matrix and increased vascular permeability leading to glomerular damage and proteinuria.

The present invention relates to compounds of formula (I) 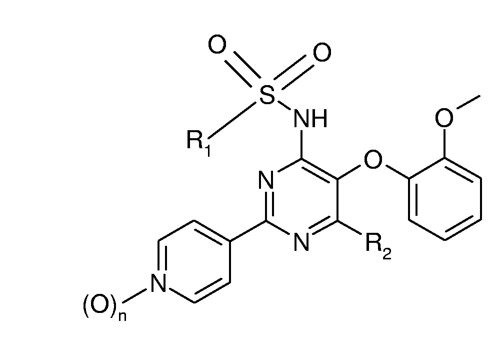 wherein
R₁ is pyridyl or thiazolyl, any of which may optionally be substituted with C₁₋₈alkyl or C₂₋₈alkenyl; and
   a) R₂ is methoxy and n is zero or one; or
   b) R₂ is chlorine and n is zero
   and pharmaceutically acceptable salts thereof.

The present invention relates to the use of a compound of formula (I) for the manufacture of a medicament for the treatment of diabetic nephropathy.

Furthermore, the present invention relates to a method of treatment of diabetic nephropathy, which comprises administering an effective diabetic nephropathy treating amount of a compound of formula (I) to a, preferably, human being or a mammalian animal.

The term "treatment" as used thoughout the description of the instant invention is meant to include also "prevention" and "delay of progression".

The sulfonamides of the present invention are known as inhibitors of endothelin receptors and a method of preparation is disclosed in WO 00/52007.

More particularly, the present invention relates to the following compounds of formula (I):
R₁ is preferably, optionally substituted with C₁₋₈alkyl or C₂₋₈alkenyl, 2-pyridyl or 2-thiazolyl and most preferably, optionally substituted with C₁₋₈alkyl or C₂₋₈alkenyl, 2-pyridyl.
C₁₋₈alkyl or C₂₋₈alkenyl are branched or straight chain radicals, for example methyl, ethyl, n-propy, isopropyl, n-butyl, isobutyl, t-butyl, vinyl, 1-propenyl, allyl, isopropenyl, 1-butenyl, 2-butenyl, 3-butenyl and the like. Preferred are said residues which have up to (and including) four carbon atoms. Most preferred is methyl.

Particularly preferred are compounds of formula (I) wherein
R₁ is 2-pyridyl optionally substituted with C₁₋₄alkyl; and
R₂ is methoxy and n is zero
and pharmaceutically acceptable salts thereof.

Most preferred is 5-methyl-pyridine-2-sulfonic acid [6-methoxy-5-(2-methoxy-phenoxy)-2-pyridin-4-yl-pyrimidin-4-yl]-amide.

The term "pharmaceutically acceptable salts" comprises salts of the compounds of formula (I) with inorganic or organic acids such as hydrochloric acid, hydrobromic acid, nitric acid, sulphuric acid, phosphoric acid, citric acid, formic acid, maleic acid, acetic acid, succinic acid, tartaric acid, methanesulphonic acid, p-toluenesulphonic acid and the like, which are nontoxic to living organisms. It also includes salts with inorganic or organic bases such as alkali salts like sodium and potassium salts, alkaline earth metal salts like calcium and magnesium salts, N-methyl-D-glutamine salts and salts with amino acids like arginine, lysine and the like.

It will be appreciated that the compounds of formula (I) in this invention may be derivatised at functional groups to provide prodrug derivatives that are capable of conversion back to the parent compounds in vivo. Additionally, any physiologically acceptable equivalents of the compounds of general formula (I), which are capable of producing the parent compounds of general formula (I) in vivo, are within the scope of this invention.

As mentioned earlier, the use of a compound of formula (I) for the manufacture of a medicament for the treatment of diabetic nephropathy is an object of the instant invention, which manufacture comprises bringing one or more compounds of formula (I) and, if desired, one or more other therapeutically valuable substances into a pharmaceutical administration form.

The pharmaceutical compositions may be administered orally, for example in the form of tablets, coated tablets, dragees, hard or soft gelatine capsules, solutions, emulsions or suspensions. Administration can also be carried out rectally, for example using suppositories; locally or percutaneously, for example using ointments, creams, gels or solutions; or parenterally, e.g. intravenously, intramuscularly, subcutaneously, intrathecally or transdermally, using for example injectable solutions. Furthermore, administration can be carried out sublingually or as opthalmological preparations or as an aerosol, for example in the form of a spray.

For the preparation of tablets, coated tablets, dragees or hard gelatine capsules the compounds of the present invention may be mixed with pharmaceutically inert, inorganic or organic excipients. Examples of suitable excipients for tablets, dragees or hard gelatine capsules include lactose, maize starch or derivatives thereof, talc or stearic acid or salts thereof.

Suitable excipients for use with soft gelatine capsules may include for example vegetable oils, waxes, fats, semi-solid or liquid polyols etc..

For the preparation of solutions and syrups, excipients which may be used include for example water, polyols, saccharose, invert sugar and glucose.

For injectable solutions, excipients which may be used include for example water, alcohols, polyols, glycerine, and vegetable oils.

For suppositories, and local or percutaneous application, excipients which may be used include for example natural or hardened oils, waxes, fats and semi-solid or liquid polyols.

### The following examples illustrate possible administration forms:

Tablets containing the following ingredients can be produced in a conventional manner:

| Ingredients | mg per tablet |
|---|---|
| Compound of formula (I) | 10.0-100.0 |
| Lactose | 125.0 |
| Corn starch | 75.0 |
| Talc | 4.0 |
| Magnesium stearate | 1.0 |

Capsules containing the following ingredients can be produced in a conventional manner:

| Ingredients | mg per capsule |
|---|---|
| Compound of formula (I) | 25.0 |
| Lactose | 150.0 |
| Corn starch | 20.0 |
| Talc | 5.0 |

Injection solutions can have the following composition:

| Compound of formula (I) | 1.0 mg |
|---|---|
| Natrium Chloride | 8.5 mg |
| Tris (hydroxymethyl) aminomethane | 0.5 mg |
| 0.1 N HCl | ad PH 8 |
| Water for injection | ad 1.0 ml |

The pharmaceutical compositions may also contain preserving agents, solubilising agents, stabilising agents, wetting agents, emulsifiers, sweeteners, colorants, odorants, salts for the variation of osmotic pressure, buffers, coating agents or antioxidants. As mentioned earlier, they may also contain other therapeutically valuable agents.

It is a prerequisite that all adjuvants used in the manufacture of the preparations are generally recognized as safe.

Preferred forms of use are intravenous, intramuscular or oral administration, most preferred is oral administration. The dosages in which the compounds of formula (I) are administered in effective amounts depend on the nature of the specific active ingredient, the age and the requirements of the patient and the mode of application. In general, dosages of about 0.01-10 mg/kg body weight per day come into consideration.

The compounds of formula (I) may also be administered in combination with antihypertensive drugs, antiarrhythmics, anti anginal, protein kinase inhibitors and/or modulators, drugs which act on proteins such as fibrinogen and matrix metalloproteinases, antithrombotics, lipid lowering agents, antioxidants and any drugs which act on the renin-angiotensin system such as angiotensin-converting enzyme inhibitors (ACEIs), renin inhibitors, and, particularly preferred, angiotensin receptor blockers (ARBs). Examples of ARBs are, among others, irbesartan, valsartan, candesartan and losartan. Said ARBs may be used at high doses in which case a high dose, by way of example, corresponds to 300 mg od irbesartan, 160 mg od valsartan, 16 mg od candesartan or 50 mg bid losartan

For above compounds which may be administered in combination with a compound of formula (I), preference is given to commercially available compounds or those compounds which have been approved by a health authority.

Consequently, a further object of the instant invention is a method of treatment of diabetic nephropathy, which comprises administering an effective diabetic nephropathy treating amount of a compound of formula (I) to a human being or a mammalian animal in combination with an ARB.

A still further object of the instant invention is a pharmaceutical composition comprising a compound of formula (I), an ARB and an excipient.

The effectiveness of the compounds of formula (I) on diabetic nephropathy can be demonstrated using the procedure described hereafter in the Example. The Example illustrates the instant invention and is not meant as limiting the invention to the embodiment specifically described.

### Example:

The study included 23 patients with diabetic nephropathy and a proteinuria higher than 500 mg/24 hours at randomisation. The study was double-blind, randomised, and placebo-controlled. All patients were treated with high-dose angiotensin receptor blockers (ARBs) for 4 weeks prior to starting treatment with 5-methyl-pyridine-2-sulfonic acid [6-methoxy-5-(2-methoxy-phenoxy)-2-pyridin-4-yl-pyrimidin-4-yl]-amide (compound A). Patients were included if at the end of the 4-week treatment period with high-dose ARBs, their 24-hour proteinuria was >300 mg/24h. They were randomised into 3 groups: 20 mg of compound A, 50 mg of compound A, or placebo once daily, on top of the high dose ARB. Treatment duration was 4 weeks. The primary variable was 24-hour proteinuria.
Out of the 23 randomised patients, 7 received compound A 20 mg, 8 received compound A 50 mg and 8 received placebo. The mean age (± SD) was similar for all 3 groups. The HbA1c level at entry was also similar for all 3 groups.

### Efficacy

The 24-hour proteinuria data show that for the individual groups, the decrease in proteinuria was -1.0 ± 1.96 g/24 h with compound A 20 mg and -1.3 ± 1.3 g/24 h with compound A 50 mg,. The placebo group showed an increase in proteinuria of 0.5 ± 1.78 g/24 h. Although there was a difference in the 24-hour proteinuria at the start of treatment with compound A among the three groups, unlike the placebo group, the 2 groups treated with compound A showed a mean decrease in 24-hour proteinuria of around 1g/24 h, which is clinically relevant.

Compound A used in above Example corresponds to 5-methyl-pyridine-2-sulfonic acid [6-methoxy-5-(2-methoxy-phenoxy)-2-pyridin-4-yl-pyrimidin-4-yl]-amide.

## Claims

1. Use of a compound of formula (I) 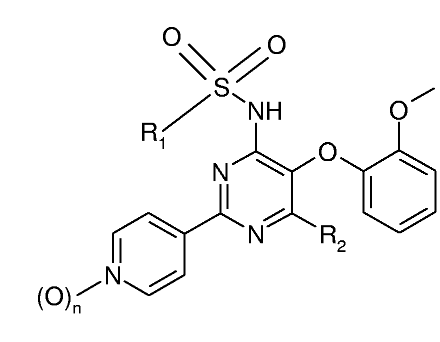 wherein
R₁ is pyridyl or thiazolyl, any of which may optionally be substituted with C₁₋₈alkyl or C₂₋₈alkenyl ; and
a) R₂ is methoxy and n is zero or one; or
b) R₂ is chlorine and n is zero
and pharmaceutically acceptable salts thereof
for the manufacture of a medicament for the treatment of diabetic nephropathy.

2. Use according to claim 1 wherein the compound of formula (I) is 5-methyl-pyridine-2-sulfonic acid [6-methoxy-5-(2-methoxy-phenoxy)-2-pyridin-4-yl-pyrimidin-4-yl]-amide.

3. A method of treatment of diabetic nephropathy, which comprises administering an effective diabetic nephropathy treating amount of a compound of formula (I) 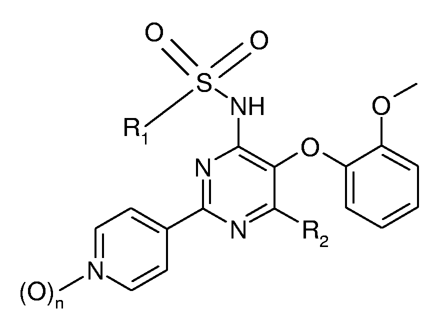 wherein
R₁ is pyridyl or thiazolyl, any of which may optionally be substituted with C₁₋₈alkyl or C₂₋₈alkenyl ; and
a) R₂ is methoxy and n is zero or one; or
b) R₂ is chlorine and n is zero
and pharmaceutically acceptable salts thereof
to a human being or a mammalian animal.

4. A method of treatment of diabetic nephropathy, which comprises administering an effective diabetic nephropathy treating amount of a compound of formula (I) 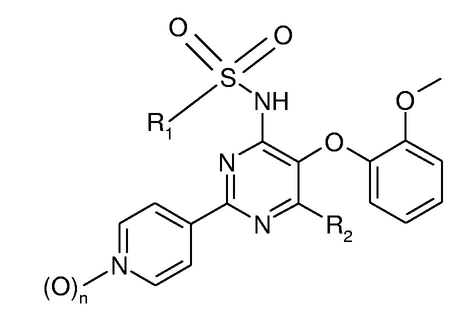 wherein
R₁ is pyridyl or thiazolyl, any of which may optionally be substituted with C₁₋₈alkyl or C₂₋₈alkenyl ; and
a) R₂ is methoxy and n is zero or one; or
b) R₂ is chlorine and n is zero
and pharmaceutically acceptable salts thereof
to a human being or a mammalian animal in combination with an ARB.

5. A method of treatment according to claim 3 or 4 wherein the compound of formula (I) is 5-methyl-pyridine-2-sulfonic acid [6-methoxy-5-(2-methoxy-phenoxy)-2-pyridin-4-yl-pyrimidin-4-yl]-amide.

6. A pharmaceutical composition comprising
A) a compound of formula (I) 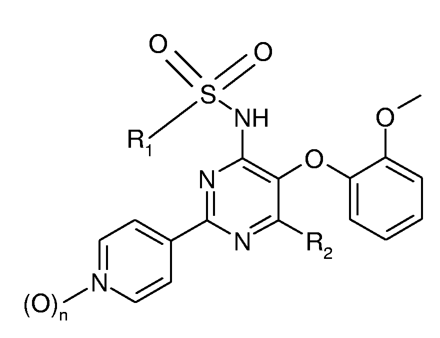 wherein
R₁ is pyridyl or thiazolyl, any of which may optionally be substituted with C₁₋₈alkyl or C₂₋₈alkenyl ; and
a) R₂ is methoxy and n is zero or one; or
b) R₂ is chlorine and n is zero
and pharmaceutically acceptable salts thereof;
B) an ARB and
C) an excipient.

7. A composition according to claim 6 wherein the compound of formula (I) is 5-methylpyridine-2-sulfonic acid [6-methoxy-5-(2-methoxy-phenoxy)-2-pyridin-4-yl-pyrimidin-4-yl]-amide.
